Europäisches Patentamt

European Patent Office

Office européen des brevets

(19)

(11) **EP 0 815 827 B1**

(12) **EUROPÄISCHE PATENTSCHRIFT**

(45) Veröffentlichungstag und Bekanntmachung des Hinweises auf die Patenterteilung:
**30.07.2003   Patentblatt 2003/31**

(51) Int Cl.$^7$: **A61K 7/06**

(21) Anmeldenummer: **97109467.7**

(22) Anmeldetag: **11.06.1997**

(54) **Kosmetisches Mittel zur Haarbehandlung mit Dendrimeren**

Hair care cosmetic composition containing dendrimers

Composition cosmétique capillaire contenant des dendrimères

(84) Benannte Vertragsstaaten:
**DE ES FR GB IT**

(30) Priorität: **28.06.1996   DE 19625982**

(43) Veröffentlichungstag der Anmeldung:
**07.01.1998   Patentblatt 1998/02**

(73) Patentinhaber: **Wella Aktiengesellschaft**
**64274 Darmstadt (DE)**

(72) Erfinder:
• **Franzke, Michael, Dr.**
**64380 Rossdorf-Gundernhausen (DE)**

• **Steinbrecht, Karin, Dr.**
**64372 Ober-Ramstadt (DE)**
• **Clausen, Thomas, Dr.**
**64665 Alsbach (DE)**
• **Schulze, Sabine**
**64572 Büttelborn (DE)**
• **TITZE, Hans-Jürgen**
**64401 Gross-Bieberau (DE)**

(56) Entgegenhaltungen:
**EP-A- 0 684 044**

## Beschreibung

Gegenstand der vorliegenden Erfindung ist ein kosmetisches Mittel zur Haarbehandlung mit einem Gehalt an mindestens einem Dendrimer oder Dendrimerkonjugat.

Ein ansprechendes äußeres Erscheinungsbild wurde schon immer als sehr wichtig angesehen. Eine besondere Rolle spielt dabei die Frisur. Basis für eine ansprechende Frisur ist gut gepflegtes Haar. Für die Reinigung und Pflege des Haares gibt es eine ganze Reihe von Haarbehandlungsmitteln wie Shampoos, Kuren, Spülungen, Spitzenfluids, die in den unterschiedlichsten Anwendungsarten z.B. als leave on- oder als rinse off--Produkte appliziert werden. Neben diesen Pflegeprodukten kommen drei weitere Produktkategorien bei der Haarveränderung zum Einsatz; nämlich permanente oder temporäre Haarfärbemittel, permanente Haarverformungsmittel in Form von mildalkalischen oder sauren Dauerwellen bzw. Haarentkrausungsmitteln sowie Mittel, die nur eine temporäre Verformung und Stabilisierung der Frisur ermöglichen und allgemein als Stylingmittel bekannt sind. Hierzu zählen Produkte wie Haarsprays, Haarlakke, Festigerlotionen, Festigerschäume, Haargele, glanzgebende Produkte, Frisurcremes etc.. Allen diesen Mitteln ist gemeinsam, daß sie in der Regel aus einer Vielzahl an Einzelsubstanzen oder Komponenten bestehen, die die unterschiedlichsten Aufgaben innerhalb der Rezeptur erfüllen.

Gesucht werden daher Substanzen, die zum einen in möglichst vielen Rezepturen verträglich sind, zum anderen je nach Formulierung unterschiedliche Funktionen übernehmen können. Von ganz besonderem Interesse sind Substanzen, die zwei oder mehrere Funktionen in einer Rezeptur übernehmen können. Zu den Forderungen, die an gepflegtes Haar gestellt werden, zählt beispielsweise, daß das Haar bereits im Nassen einen guten, angenehmen Griff und eine gute Kämmbarkeit haben sollte. Im Trockenen werden ebenfalls ein angenehmer Griff, Elastizität und Volumen (Ausnahme "Wetlook" Frisuren) erwartet.

Die Aufgabe dieser Erfindung bestand also darin, eine neue Substanzklasse zu finden, die die oben genannten Anforderungen erfüllt. Eine solche Substanzklasse wurde mit der im folgenden näher erläuterten Substanzklasse der Starburst-Dendrimeren gefunden. Dendrimere sind in der gesamten Breite der Haarbehandlungsmittel einsetzbar.

Schon die niedermolekularen Dendrimere bewirken in saurem Milieu eine deutliche Reduzierung der Naßkammkräfte sowie einen guten Griff. Das so behandelte trockene Haar hat gutes Volumen und Elastizität.

Gegenstand der vorliegenden Erfindung ist daher ein kosmetisches Mittel zur Haarbehandlung mit einem Gehalt an mindestens einem Dendrimer oder Dendrimerkonjugat in einer geeigneten kosmetischen Grundlage.

Dendrimere sind dreidimensionale, hochverzweigte Oligomere und Polymere mit einer eindeutig definierten chemischen Struktur. Im allgemeinen bestehen Dendrimere aus einem Kern, aus einer Anzahl an Verzweigungen, den sogenannten Generationen, und aus den Endgruppen.

Dendrimere werden in einer Reihe von Patentanmeldungen beschrieben. So sind in der US 5,210,309 Bausteine beschrieben, aus denen Dendrimere hergestellt werden können. Die US-4,102,827 und die US-4,036,808 beschreiben die Herstellung hochkationischer Polyelektrolyte in Form von verzweigten Polyelektrolyten oder sternförmigen Polyelektrolyten sowie den Einsatz solcher kationischer Elektrolyte in leitfähigen Hydrogelen. In der WO 93/21144 wird die Herstellung von Kaskadenpolymeren beschrieben. Eine Reihe von Anmeldungen beschreibt die Herstellung von Dendrimeren und ihre Anwendung für pharmazeutische Zwecke. In der EP 0 271 180 werden sternförmige Konjugate für pharmazeutische und agrokulturelle Zwecke beschrieben. In der SE 468 771 wurde die Herstellung von Polyester-Dendrimeren und ihre Anwendung als Kupplungskomponenten in Polyester, Epoxy- oder Polyurethanharzen sowie ihr Einsatz als Binderkomponente für eine Reihe nicht-kosmetischer Anwendungen beansprucht. Die WO 92/14543 beschreibt unimolekulare Micellen auf Basis von Kaskadenpolymeren und ihre Herstellung. In der US-4,587,329 und in der US-4,558,120 wird die Herstellung von sternförmigen Dendrimeren und ihre Anwendung als demulsifier in O/W-Emulsionen beschrieben. In der EP 0 247 629 wird der Einsatz von sternförmigen Dendrimeren zur Calibrierung und Charakterisierung von Substanzen mit Ausmaßen bis zu 10μm beansprucht. Neben diesen in Patenten beschriebenen Dendrimeren existieren noch eine Reihe von wissenschaftlichen Publikationen zur Herstellung von Dendrimeren. Dendrimere können auf sehr unterschiedlicher Art und Weise hergestellt werden. So kann die Herstellung nach den in der WO 93/21144 oder der EP 0 271 180 beschriebenen Verfahren erfolgen. weitere mögliche Herstellungsverfahren sind in der US-4,587,329, der US-4,558,120 sowie der WO 92/14543 beschrieben. Daneben existieren eine Reihe von weiteren Veröffentlichungen, in denen die Herstellung von Dendrimeren beschrieben wird.

Das erfindungsgemäße kosmetische Mittel enthält bevorzugt Dendrimere, welche aus dem Kern 1,4-Diaminobutan über eine stufenweise ablaufende Michael-Addition von Acrylnitril mit anschließender katalytischer Hydrierung der Cyanogruppen zu Aminogruppen hergestellt werden, wobei Dendrimere mit Nitriloder Aminoendgruppen erhalten werden. Diese Dendrimere werden auch als Poly(iminopropan-1,3-diyl)dendrimere mit Nitril- bzw. Aminoendgruppen bezeichnet. Geeignete Dendrimere werden beispielsweise unter der Bezeichnung 4-Cascade: 1,4-Diaminobutan [4]: propylamin bzw. x-Cascade:1,4-Diaminobutan [4]:(1-azabutyliden)$^{x-4}$:propylamin von der Firma DSM/Niederlande vertrieben, wobei x die Werte 4, 8, 16, 32 oder 64 annehmen kann.

Das erfindungsgemäße kosmetische Mittel enthält bevorzugt 0,01 bis 75 Gewichtsprozent, besonders bevorzugt 0,1 bis 35 Gewichtsprozent mindestens eines Dendrimers oder Dendrimerkonjugats. Das erfindungsgemäße Mittel kann in Form von Haarwaschmitteln, Haarpflege-, Haarfestigungs, Haarfärbe- oder Tönungsmittel, Blondiermittel sowie als Dauerwellmittel vorliegen. Das Mittel kann als Lotion, Schaum, Milch, Gel, Creme, Gelschaum, Spray appliziert werden. Es kann als leave on - wie auch als rinse off-Produkt formuliert werden.

Das erfindungsgemäße Mittel kann darüber hinaus für Haarbehandlungsmittel übliche Zusatzbestandteile enthalten, zum Beispiel Lösungsmittel, wie Wasser und niedere aliphatische Alkohole, beispielsweise Ethanol, Propanol und Isopropanol, oder Glykole wie Glycerin und 1,2-Propylenglykol; Netzmittel oder Emulgatoren aus den Klassen der anionischen, kationischen, amphoteren oder nichtionogenen oberflächenaktiven Tenside, wie Fettalkoholsulfate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, in einer Menge von 0,1 bis 30 Gewichtsprozent; Parfümöle in einer Menge von 0,1 bis 5,0 Gewichtsprozent; Trübungsmittel, wie zum Beispiel Ethylenglykoldistearat, in einer Menge von etwa 0,2 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10 Gewichtsprozent; bakterizide und fungizide Wirkstoffe, wie zum Beispiel 2,4,4-Trichlor-2-hydroxydiphenylether oder Methylchlorisothiazolion, in einer Menge von 0,01 bis 1,0 Gewichtsprozent; Verdickungsmittel, wie beispielsweise Kokosfettsäurediethanolamid, in einer Menge von etwa 0,2 bis 3,0 Gewichtsprozent; Puffersubstanzen, wie beispielsweise Natriumcitrat oder Natriumphosphat, in einer Menge von 0,1 bis 1,0 Gewichtsprozent; Lösungsvermittler, wie zum Beispiel ethoxyliertes Rizinusöl, in einer Menge von etwa 0,1 bis 1,0 Gewichtsprozent; Anfärbestoffe, wie zum Beispiel Fluorescein-Natriumsalz, in einer Menge von etwa 0,1 bis 1,0 Gewichtsprozent; Pflegestoffe, wie zum Beispiel Pflanzen- und Kräuterextrakte, Protein- und Seidenhydrolysate, kationische Harze, Lanolinderivate, in einer Menge von 0,1 bis 5 Gewichtsprozent; physiologisch verträgliche Silikonderivate, wie zum Beispiel Silikonöl, Silikonpolymere und Siloxane; Lichtschutzmittel, Feuchthaltemittel, Antioxidantien, Radikalfänger, Antischuppenwirkstoffe, in einer Menge von etwa 0,01 bis 2 Gewichtsprozent; physiologisch verträgliche organische oder anorganische Säuren, wie zum Beispiel Ameisensäure, Glyoxylsäure, Milchsäure, Weinsäure, Zitronensäure oder Phosphorsäure, natürliche, modifizierte natürliche oder synthetische Polymere, wie zu Beispiel Schellack, kationische, anionische, nichtionische, amphotere Polymere, Chitosan, Chitin oder Chitosanderivate; direktziehende Haarfarbstoffe, Haarfarbstoffe, die oxidativ entwickelt werden, Oxidationsmittel, Reduktionsmittel, Fettalkohole, Glanzgeber, Vitamine, Weichmacher, Kämmbarkeitsverbesserer, rückfettende Agentien, Entschäumer sowie Treibgase, wie zum Beispiel Fluorkohlenwasserstoffe, Dimethylether, Kohlenwasserstoffe und kom primierbare Gase.

In einer bevorzugten Ausführungsform enthält das erfindungsgemäße Mittel zusätzlich 0,01 bis 40 Gewichtsprozent, besonders bevorzugt 0,01 bis 25 Gewichtsprozent mindestens eines anionischen, kationischen, amphoteren oder nicht-ionischen Tensids und 50 bis 90 Gewichtsprozent Wasser. Ein derartiges Mittel kann als Haarreinigungsmittel verwendet werden.

In einer weiteren, bevorzugten Ausführungsform enthält das erfindungsgemäße Mittel zusätzlich mindestens ein natürliches oder synthetisches Polymer, welches ausgewählt ist aus den Gruppen der festigenden und der verdikkenden Polymere. Die Polymere können in Mengen von 0,01 bis 25 Gewichtsprozent, bevorzugt 0,1 bis 20 Gewichtsprozent eingesetzt werden und in gelöster Form oder als Dispersion vorliegen. Ein derartiges Mittel kann bei Einsatz von festigenden Polymeren als Haarfestigungsmittel verwendet werden.

Wenn das erfindungsgemäße Mittel als Mittel zur dauerhaften Verformung des Haares vorliegt, enthält es zusätzlich 0,5 bis 15 Gewichtsprozent mindestens einer keratinreduzierenden Mercaptoverbindung und liegt bevorzugt als wäßrige, alkalisch oder schwach sauer (pH = 5 bis 10) eingestellte Zubereitung vor, welche als keratinreduzierende Mercaptoverbindung, beispielsweise Cystein, Cysteamin, N-Acetyl-L-Cystein, Mercaptocarbonsäure, wie zum Beispiel Thioglykolsäure oder Thiomilchsäure, oder Salze von Mercaptocarbonsäuren, wie zum Beispiel Ammonium- und Guanidinsalze der Thioglykolsäure oder Thiomilchsäure enthält.

Die erforderliche Alkalität wird hierbei durch die Zugabe von Ammoniak, organischen Aminen, Ammonium- und Alkalicarbonaten oder -hydrogencarbonaten eingestellt. Es kommt aber auch ein neutral oder sauer (pH = 4,5 bis 7) eingestelltes Haarverformungsmittel in Betracht, das im wäßrigen Medium einen wirksamen Gehalt an Sulfiten oder Mercaptocarbonsäureesternaufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als $SO_2$) verwendet:. Im letzteren Fall kommen insbesondere Thioglykolsäuremonoglykolester oder -glycerinester in einer Konzentration von etwa 5 bis 50 Gewichtsprozent (entsprechend einem Gehalt an freier Thioglykolsäure von 2 bis 16 Gewichtsprozent) zur Anwendung.

Das erfindungsgemäße Mittel zur dauerhaften Haarverformung kann auch ein Gemisch der vorstehend genannten keratinreduzierenden Verbindungen enthalten.

Nach einer für die dauerhafte Verformung des Haares ausreichenden Einwirkungszeit, welche, je nach Haarbeschaffenheit, dem pH-Wert und der Verformungswirksamkeit des Verformungsmittels sowie in Abhängigkeit von der Anwendungstemperatur, etwa 10 bis 30 Minuten beträgt, wird das Haar mit wasser gespült und anschließend oxidativ nachbehandelt ("fixiert"). Das Nachbehandlungsmittel wird, je nach Haarfülle, in einer Menge von etwa 50 bis 100 g verwendet.

Für die oxidative Nachbehandlung kann ein erfindungsgemäßes Fixiermittel mit einem Gehalt an Dendrimeren oder Dendrimerkonjugaten oder jedes beliebige bisher für eine derartige Behandlung verwendete Fixiermittel verwendet werden. Beispiele für in einem derartigen Fixiermittel verwendete Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Üblicherweise liegt das Oxidationsmittel in dem wäßrigen Fixiermittel in einer Konzentration von etwa 0,5 bis 10 Gewichtsprozent vor.

Sowohl das erfindungsgemäße Mittel zur dauerhaften Haarverformung als auch das erfindungsgemäße Fixiermittel kann in Form einer wäßrigen Lösung oder Emulsion sowie in verdeckter Form auf wäßriger Basis, insbesondere als Creme, Gel oder Paste vorliegen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Das vorstehend beschriebene Verfahren zur dauerhaften Haarverformung ermöglicht eine schonende und gleichmäßige Umformung vom Haaransatz bis zu den Haarspitzen, das Haar zeigt eine hervorragende Naß- und Trockenkämmbarkeit, einen angenehmen Griff und einen ansprechenden Glanz im getrockneten Zustand sowie eine lockere, sprunghafte und gleichmäßige Dauerwellung, insbesondere im Bereich der Haarspitzen.

Ein erfindungsgemäßes Haartönungsmittel enthält zusätzlich 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C.I. 14 805), Anthrachinonfarbstoffe, zum Beispiel Disperse Violet 4 (C. I. 61 105), Triphenylmethanfarbstoffe, zum Beispiel Basic Violet 1 (C. I. 42 535), wobei die Farbstoffe je nach Art ihrer Substituenten sauren, nichtionogenen oder basischen Charakter haben können und/oder natürlichen Haarfarbstoffs, wie zum Beispiel Henna oder Reng, der zur Farbentwicklung nicht der Oxidation bedarf.

Das erfindungsgemäße kosmetische Mittel kann unter Verwendung eines Treibmittels oder mit Hilfe einer mechanisch betriebenen Sprühvorrichtung versprüht werden oder mit Hilfe einer Schaumerzeugungsvorrichtung als Schaum abgegeben werden.

Wenn das erfindungsgemäße kosmetische Mittel mit Hilfe eines Treibmittels versprüht wird, so enthält es bevorzugt 3 bis 75 Gewichtsprozent des Treibmittels und wird in einem Druckbehälter abgefüllt.

Als Treibmittel sind beispielsweise niedere Alkane, wie zum Beispiel n-Butan, i-Butan und Propan oder deren Gemische oder auch Dimethylether und Fluorkohlenwasserstoffe, wie beispielsweise F 152 (1,1-Difluorethan) oder F 134 (Tetrafluorethan) sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet. Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem Mittel infolge der Kontraktion des elastischen Behälters beim Öffnen des Sprühventils kontinuierlich abgegeben wird, verwendet werden. Als geeignete mechanische Schaumerzeugungsvorrichtung kann beispielsweise der in der EP-B 0 0460 154 beschriebene Aufsatz mit einer Schaumerzeugungseinrichtung auf einem flexiblen Behälter verwendet werden.

Wird das erfindungsgemäße kosmetische Mittel zur Haarpflege eingesetzt, so wird es folgendermaßen angewandt:

Nach der Haarwäsche werden in dem handtuchtrockenen Haar, je nach Haarfülle, 5 bis 30 g des Mittels verteilt und ca. 3 bis 15 Minuten einwirken gelassen. Anschließend wird das Mittel ausgespült, das Haar durchgekämmt, gegebenenfalls zur Frisur geformt und getrocknet. Eine bevorzugte Ausführung des erfindungsgemäßen Mittels erlaubt den Verbleib im Haar, das heißt es wird nicht ausgespült und erspart dem Anwender dadurch einen Arbeitsgang.

Wenn das erfindungsgemäße Mittel als Oxidationshaarfärbemittel vorliegt, so enthält es zusätzlich zu dem Dendrimer oder Dendrimerkonjugat 0,01 bis 5,0 Gewichtsprozent einer geeigneten Kupplersubstanz und 0,01 bis 5,0 Gewichtsprozent einer geeigneten Entwicklersubstanz. Geeignete Kupplersubstanzen sind beispielsweise Resorcin, 4-Chlorresorcin, 1-Naphtol, 3-Aminophenol oder Derivate des m-Phenylendiamins. Geeignete Entwicklersubstanzen sind beispielsweise 2,5-Diaminotoluol, 2,5-Diaminophenylethylalkohol, p-Aminophenol oder 1,4-Diaminobenzol. Zur Entwicklung der Haarfärbung wird ein geeignetes Oxidationsmittel wie beispielsweise Wasserstoffperoxid oder dessen Additionsverbindungen an Harnstoff, Melamin, Natriumborat oder Natriumcarbonat in Form einer 3-bis 12prozentigen wäßrigen Lösung aber auch Luftsauerstoff verwendet.

Die nachfolgenden Beispiele sollen den Gegenstand der Erfindung näher erläutern.

**Beispiele:**

**Beispiel 1: Dauerwellverformungsmittel**

| A | B | |
|---|---|---|
| 7,4 g | -- | 4-Cascade: 1,4-Diaminobutan [41:-propylamin |
| -- | 10, 0 g | 32-Cascade: 1,4-Diaminobutan [4]:-(1-azabutyliden)$^{28}$: propylamin |
| 8,0 g | 8,0 g | Thioglykolsäure |
| 2,6 g | 2,6 g | Ammoniumhydrogencarbonat |
| 0,3 g | 0,3 g | Glycerin-polyethylenglykol-(35)-rizinoleat |
| 0,3 g | 0,3 g | Parfüm |
| 0,1 g | 0,1 g | Octylphenol, oxethyliert mit 20 Mol Ethylenoxid |
| 81,3 g | 78,7 g | Wasser |
| 100,0 g | 100,0 g | |
| 8,1 | 8,1 | pH-Wert |

**Beispiel 2: Dauerwellverformungsmittel**

| | |
|---|---|
| 7,36 g | 8-Cascade: 1,4-Diaminobutan [4]-(1-azabutyliden)$^{4}$:propylamin |
| 7,06 g | Thiomilchsäure |
| 2,50 g | L-Cystein |
| 1,50 g | Polyproylen-(1)-Polyethylen-(9)-laurylglykolether |
| 0,75 g | Parfüm |
| 0,23 g | Dimethyldiallylammoniumchlorid |
| 80,60 g | Wasser |
| 100,00 g | |
| 8,4 | pH-Wert |

**Beispiel 3: Saures Dauerwellverformungsmittel**

| | |
|---|---|
| 0,55 g | 16-Cascade:1,4-Diaminobutan[4]:(1-azabutyliden)$^{12}$: propylamin |
| 7,28 g | Monothioglykolsäureglycerinester |
| 0,71 g | DL-2-Pyrrolidon-5-carbonsäure |
| 0,60 g | Parfüm |
| 0,50 g | Glycerinpolyethylenglykol-(35)-rizinoleat |
| 0,40 g | Octylphenol, ethoxyliert mit 20 Mol Ethylenoxid |
| 0,30 g | Vinyipyrrolidon/Methacrylamidopropyltrimethylammoniumchlorid Copolymer |
| 0,20 g | Vinylpyrrolidon/Polystyrol Copolymer |
| 89,46 g | Wasser |
| 100,00 g | |
| 6,5 | pH-Wert |

**Beispiel 4: Dauerwell-Fixiermittel**

| | |
|---|---|
| 0,5 g | 8-Cascade: 1,4-Diaminobutan [4]:(1-azabutyliden)$^{4}$:propylamin |

(fortgesetzt)

| 10,0 g | Natriumbromat |
|---|---|
| 3,2 g | Dinatriumhydrogenphosphat Dodecahydrat |
| 0,8 g | Ortho-Phosphorsäure (85prozentig) |
| 0,5 g | Mononatriumphosphat |
| 85,0 g | Wasser |
| 100,0 g | |
| 6,4 | pH-Wert |

**Beispiel 5: Schaumfixiermittel**

| 1,15 g | 4-Cascade: 1,4-Diaminobutan [4]: propylamin |
|---|---|
| 14,0 g | Wasserstoffperoxid (35prozentig) |
| 3,41 g | o-Phosphorsäure (85prozentig) |
| 0,8 g | Ortho-Phosphorsäure |
| 1,25 g | DL-2-Pyrrolidon-5-carbonsäure |
| 0,66 g | Laurylaminodimethylacetobetain |
| 0,50 g | Polyropylen-(1)-polyethylen-(9)-lauryk-glykolether |
| 0,20 g | Parfüm |
| 0,05 g | p-Acetaminophenol |
| 78,78 g | Wasser |
| 100,0 g | |
| 2,2 | pH-Wert |

**Beispiel 6: Fixiermittel**

| 1,02 g | 32-Cascade: 1,4-Diaminobutan [4]: (1-azabutyliden)[23]: propylamin |
|---|---|
| 20,00 g | Wasserstoffperoxid |
| 3,05 g | o-Phosphorsäure (85prozentig) |
| 1,50 g | Dinatriumhydrogenphosphat |
| 1,13 g | Cetyltrimethylammoniumchlorid |
| 0,45 g | Hydriertes Rhizinusöl, ethoxyliert mit 45 Mol Ethylenoxid |
| 0,35 g | DL-2-Pyrrolidon-5-carbonsäure |
| 0,30 g | Parfüm |
| 0,05 g | 1-Hydroxy-ethyliden-1,1-diphosphonsäure |
| 72,15 g | Wasser |
| 100,00 g | |
| 2,2 | pH-Wert |

**Beispiel 7: Shampoo**

| 0,60 g | 16-Cascade: 1,4-Diaminobutan [4]:(1-aza-butyliden)[12]: propylamin |
|---|---|
| 15,90 g | Decylpolyglucose |
| 3,00 g | Kokosfettsäureamidopropylbetain |
| 2,45 g | Polyethylenglykol-(120)-methylglucose-dioleat |
| 0,95 g | Zitronensäure |

(fortgesetzt)

| 0,50 g | Parfüm |
|---|---|
| 0,35 g | Natriumbenzoat |
| 0,06 g | Natriumformiat |
| 76.19 g | Wasser Wasser |
| 100,00 g | |
| 5,6 | pH-Wert |

**Beispiel 8: Shampoo**

| 0,30 g | 4-Cascade: 1,4-Diaminobutan [4]: propylamin |
|---|---|
| 11,62 g | Laurylethersulfat |
| 3,00 g | Natriumchlorid |
| 0,37 g | Zitronensäure |
| 0,20 g | Parfüm |
| 0,06 g | 1,2-Dibrom-2,4-dicyanobutan |
| 87,45 g | Wasser |
| 100,00 g | |
| 5,3 | pH-Wert |

**Beispiel 9: Konditionierendes Shampoo**

| 0,80 g | 32-Cascade: 1,4-Diaminobutan [4]:(1-azabutyliden )[28] :propylamin |
|---|---|
| 12,00 g | Laurylaminodimethylacetobetain |
| 6,40 g | Kaliumcocoyl-Kollagenhydrolysat |
| 3,50 g | Kokosfettsäuremonoethanolamid |
| 1,30 g | DL-2-Pyrrolidon-5-carbonsäure |
| 0,88 g | Kollagenhydrolysat |
| 0,30 g | Parfüm |
| 0,23 g | Zitronensäure |
| 0,15 g | 1,2-Dibrom-2,4-dicyanobutan |
| 74,44 g | Wasser |
| 100,00 g | |
| 5,4 | pH-Wert |

**Beispiel 10: Antifett-Shampoo**

| 0,50 g | 4-Cascade: 1,4-Diaminobutan [4]: propylamin |
|---|---|
| 11,20 g | Laurylethersulfat |
| 5,00 g | Pflanzenextrakt Extrapon Efeu der Firma Dragoco/Deutschland |
| 3,00 g | Pflanzenextrakt Extrapon Vanille Spezial der Firma Dragoco/Deutschland |
| 3,00 g | Kokosfettsäureamidopropylbetain |
| 2,00 g | Natriumchlorid |
| 1,70 g | 1,2-Propylenglykol |
| 1,10 g | DL-2-Pyrrolidon-5-carbonsäure |
| 0,30 g | p-Hydroxybenzoesäuremethylester |

(fortgesetzt)

| | |
|---|---|
| 0,15 g | Parfüm |
| 72,05 g | Wasser |
| 100,00 g | |
| 5,3 | pH-Wert |

**Beispiel 11: Antischuppen-Shampoo**

| | |
|---|---|
| 0,65 g | 64-Cascade: 1,4-Diaminobutan [4]:(1-azabutyliden) 60: propylamin |
| 25,00 g | Monocarboxymethyl-cocosimidazolin |
| 6,00 g | 3-[(3-Cocoamidopropyl )dimethylammonio]2-hydroxypropansulfonat |
| 3,00 g | Glycerylstearat |
| 1,75 g | Polyethylenglykol-(120)-methylglucosedioleat |
| 1,20 g | Ameisensäure |
| 1,00 g | 1,2-Propylenglykol |
| 0,40 g | kationische Hydroxyethylcellulose |
| 0,40 g | 1-Hydroxy-4-methyl-6-(2,4,4-trimethyl-pentyl)-2(1H)-pyridon Monoethanolaminsalz |
| 0,30 g | Parfüm |
| 0,15 g | Ethylendiamintetraacetat, Natriumsalz |
| 0,15 g | Wasser |
| 100,00 g | |
| 4,9 | pH-Wert |

**Beispiel 12: Two-in-One Shampoo**

| | |
|---|---|
| 0,71 g | 8-Cascade: 1,4-Diaminobutan [4]:(1-aza-butyliden)$^4$:propylamin |
| 16,52 g | Lauryldimethylcarboxymethylammoniumbetain |
| 5,00 g | Dioxyethylenlaurylether |
| 4,00 g | Tensid Rewoderm 66 E 10 der Firma Rewo/Deutschland |
| 2,20 g | Laurinsäurediethanolamid |
| 2,00 g | Diquaternäres Polydimethylsiloxan (Abil Quat 3272 der Firma Goldschmidt:/Deutschland |
| 0,71 g | Ameisensäure |
| 0,40 g | Parfüm |
| 0,15 g | 1,2-Dibrom-2,4-dicyanobutan |
| 68,31 g | Wasser |
| 100,00 g | |
| 5,2 | pH-Wert |

**Beispiel 13: Haarkurmittel**

| | |
|---|---|
| 0,93 g | 32-Cascade: 1,4-Diaminobutan [4]:(1-azabutyliden )$^{28}$ :propylamin |
| 5,00 g | Glycerinmonodistearat |
| 4,00 g | Stearylalkohol |
| 2,07 g | Zitronensäure |
| 1,13 g | Cetyltrimethylammoniumchlorid |
| 0,50 g | Glycerin (86prozentig) |

(fortgesetzt)

| | |
|---|---|
| 0,50 g | Pflanzenextrakt Extrapon Bambus der Firma Dragoco/Deutschland |
| 0,30 g | Parfüm |
| 85,57 g | Wasser |
| 100,00 g | |
| 3,4 | pH-wert |

## Beispiel 14: Intensivhaarkur

| | |
|---|---|
| 0,60 g | 64-Cascade:1,4-Diaminobutan[4]:(1-azabutyliden)$^{60}$: propylamin |
| 6,00 g | Cetylstearylalkohol |
| 2,00 g | Wollfett |
| 1,50 g | DL-2-Pyrrolidon-5-carbonsäure |
| 1,00 g | Cetyltrimethylammoniumchlorid |
| 0,83 g | Distearyldimethylammoniumchlorid |
| 0,60 g | 1,2-Propylenglykol |
| 0,20 g | Parfüm |
| 0,18 g | Kollagenhydrolysat |
| 87,09 g | Wasser |
| 100,00 g | |
| 3,2 | pH-Wert |

## Beispiel 15: Haarbalsam

| | |
|---|---|
| 0,50 g | 16-Cascade:1,4-Diaminobutan[4]:(1-azabutyliden)$^{12}$: propylamin |
| 6,00 g | Glycerylstearat/Polyethylenglykol-(20)cetearylether |
| 4,00 g | Diquaternäres Polydimethylsiloxan (Abile Quat 3272 der Firma Goldschmidt/Deutschland) |
| 2,00 g | Cetylalkohol |
| 1,36 g | Zitronensäure |
| 0,14 g | 1,2-Dibrom-2,4-dicyanobutan |
| 0,12 g | Parfüm |
| 85,88 g | Wasser |
| 100,00 g | |
| 3,5 | pH-Wert |

## Beispiel 16: HaarspÜlung

| | |
|---|---|
| 0,75 g | 8-Cascade: 1,4-Diaminobutan [4]:(1-azabutyliden )$^{4}$: propylamin |
| 4,00 g | Cetylstearylalkohol |
| 1,36 g | DL-2-Pyrrolidon-5-carbonsäure |
| 0,75 g | Cetyltrimethylammoniumchlorid |
| 0,50 g | Parfüm |
| 0,20 g | Pflanzenextrakt Extrapon 5 Spezial der Firma Dragoco/Deutschland |
| 92,44 g | Wasser |
| 100,00 g | |
| 4,7 | pH-Wert |

**Beispiel 17: Versprühbares Haarkurmittel**

| | |
|---|---|
| 0,40 g | 32-Cascade: 1,4-Diaminobutan [4]:(1-azabutyliden )[28] :propylamin |
| 2,00 g | Dimethyldiallylammoniumchlorid |
| 1,25 g | Polyethylenglykol-(40)-sorbitanmonopalmitat |
| 1,00 g | DL-2-Pyrrolidon-5-carbonsäure |
| 0,10 g | Parfüm |
| 0,03 g | Cetyltrimethylammoniumchlorid |
| 15,00 g | Ethanol |
| 80,22 g | Wasser |
| 100,00 g | |
| 4,2 | pH-Wert |

**Beispiel 18: Schaumförmiges Haarkurmittel**

| | |
|---|---|
| 0,80 g | 4-Cascade: 1,4-Diaminobutan [4]: propylamin |
| 2,00 g | Kationische Emulsion von aminfunktionalisiertem Polydimethylsiloxan (929 Cationic Emulsion der Firma Dow Corning Europe/-Belgien) |
| 1,30 g | Zitronensäure |
| 0,50 g | Hydroxypropylcellulose (M = 1.150.000 g/mol) |
| 0,30 g | Silikonwachs (2501 Cosmetic Wax der Firma Dow Corning Europe/Belgien) |
| 0,20 g | Parfüm |
| 0,25 g | Cetyltrimethylammoniumchlorid |
| 0,15 g | D-Panthenol |
| 0,10 g | Elastinhydrolysat |
| 5,00 g | Propan/Butan (5,0 bar) |
| 10,00 g | Ethanol |
| 79,40 g | Wasser |
| 100,00 g | |
| 5,2 | pH-Wert |

**Beispiel 19: Haarfestigungsmittel**

| | |
|---|---|
| 0,50 g | 32-Cascade:1,4-Diaminobutan[4]-.(1-azabutyliden)[28] :propylamin |
| 3,00 g | Vinylpyrrolidon/Vinylacetat Copolymer |
| 0,90 g | Ameisensäure |
| 0,20 g | 1,2-Propylenglykol |
| 0,15 g | Parfüm |
| 0,03 g | Cetyltrimethylammoniumchlorid |
| 20,00 g | Wasser |
| 75,22 g | Ethanol |
| 100,00 g | |
| 4,7 | pH-Wert |

**Beispiel 20: Fönlotion mit UV-Schutz**

| | |
|---|---|
| 0,60 g | 16-Cascade: 1,4-Diaminobutan [4]:(1-aza-butyliden)$^{12}$: propylamin |
| 2,15 g | DL-2-Pyrrolidon-S-carbonsäure |
| 1,50 g | Vinylpyrrolidon/Vinylacetat Copolymer |
| 1,25 g | Vinylpyrrolidon/Dimethylaminoethylmethacrylat Copolymer |
| 0,20 g | Parfüm |
| 0,15 g | Glycerin (85prozentig) |
| 0,10 g | 2-Hydroxy-4-methoxybenzophenon |
| 42,80 g | Wasser |
| 51,25 g | Ethanol |
| 100,00 g | |
| 4,3 | pH-Wert |

**Beispiel 21: Haarpflegende Festigerlotion**

| | |
|---|---|
| 0,75 g | 64-Cascade:1,4-Diaminobutan[4]:(1-azabutyliden)$^{60}$: propylamin |
| 4,00 g | Vinylpyrrolidon/Vinylacetat Copolymer |
| 1,20 g | DL-2-Pyrrolidon-5-carbonsäure |
| 0,40 g | Hydriertes Rhizinusöl, ethoxyliert mit 40 Mol Ethylenoxid |
| 0,20 g | Parfüm |
| 93,45 g | Wasser |
| 100,00 g | |
| 3,8 | pH-Wert |

**Beispiel 22: Schaumfestiger mit starker Festigung**

| | |
|---|---|
| 0,30 g | 64-Cascade: 1,4-Diaminobutan [4] - (1-azabutyliden)$^{60}$: propylamin |
| 5,00 g | Vinylpyrrolidon/Vinylacetat Copolymer |
| 0,60 g | DL-2-Pyrrolidon-S-carbonsäure |
| 0,45 g | Glyceryllaurat |
| 0,15 g | Parfüm |
| 0,06 g | Cetyltrimethylammoniumchlorid |
| 5,00 g | Propan/Butan (5,0 bar) |
| 10,00 g | Ethanol |
| 78,44 g | Wasser |
| 100,00 g | |
| 3,8 | pH-Wert |

**Beispiel 23: Schaumfestiger**

| | |
|---|---|
| 0,60 g | 8-Cascade: 1,4-Diaminobutan [4] : (1-aza-utyliden)$^{4}$:propylamin |
| 1,80 g | Chitosan |
| 1,10 g | Ameisensäure |
| 0,20 g | 1,2-Propylenglykol |

(fortgesetzt)

| | |
|---|---|
| 0,20 g | Parfüm |
| 0,10 g | Cetyltrimethylammoniumchlorid |
| 6,00 g | Propan/Butan (5,0 bar) |
| 10,00 g | Ethanol |
| 80,00 g | Wasser |
| 100,00 g | |
| 4,1 | pH-Wert |

**Beispiel 24: Schaumfestiger**

| | |
|---|---|
| 0,70 g | 16-Cascade: 1,4-Diaminobutan [4]:(1-azabutyliden): propylamin |
| 3,15 g | Polyvinylpyrrolidon |
| 1,60 g | Zitronensäure |
| 0,60 g | Hydriertes Rhizinusöl, ethoxyliert mit 45 Mol Ethylenoxid |
| 0,22 g | Decylpolyglucosid |
| 0,20 g | Vinylpyrroiidon/Methacrylamidopropyltrimethylanimoniumchlorid Copolymer |
| 0,20 g | Parfüm |
| 6,00 g | Propan/Butan (5,0 bar) |
| 87,33 g | Wasser |
| 100,00 g | |
| 3,7 | pH-Wert |

**Beispiel 25: Lockenschaumfestiger**

| | |
|---|---|
| 0,50 g | 4-Cascade: 1,4-Diaminobutan [4]: propylamin |
| 2,20 g | Vinylimidazoliummethochlorid/1-Vinyl-2-Pyrrolidon-Copolymer |
| 1,00 g | Glukosesirup |
| 0,70 g | Oleyl-polyethylenglykol-(200)-ether |
| 0,60 g | Zitronensäure |
| 0,10 g | Parfüm |
| 7,00 g | Propan/Butan (5,0 bar) |
| 10,00 g | Ethanol |
| 77,90 g | Wasser |
| 100,00 g | |
| 6,3 | pH-Wert |

**Beispiel 26: Pflegender Schaumfestiger**

| | |
|---|---|
| 0,8 g | 32-Cascade:1,4-Diaminobutan[4]:(1-azabutyliden)[28]: propylamin |
| 6,0 g | Vinylcaprolactam/vinylpyrrolidon/Dimethylaminoethylmethacrylat Terpolymer |
| 0,6 g | Ameisensäure |
| 0,2 g | Cetyltrimethylammoniumchlorid |
| 0,2 g | Hydriertes Rhizinusöl, ethoxyliert mit 45 Mol Ethylenoxid |
| 0,2 g | Parfüm |
| 92,0 g | Wasser |

(fortgesetzt)

| 100,0 g | |
| --- | --- |
| 4,4 | pH-Wert |

Die Mischung wird im Verhältnis 94:6 mit einem Propan/-Butan-Treibgasgemisch abgefüllt.

**Beispiel 27: Farbfestiger**

| 0,23 g | 8-Cascade: 1,4-Diaminobutan [4]:(1-azabutyliden)[4]:propylamin |
| --- | --- |
| 0,50 g | Vinylacetat/Crotonsäure/Polyglykol Copolymer |
| 0,20 g | Parfüm |
| 0,07 g | 1-Amino-4-(2',3'-dehydroxypropyl)amino-5-chlor-2-nitrobenzol |
| 0,05 g | Basic Brown 17 (C. I. 12 251) |
| 0,01 g | Basic Blue 7 (C. I. 42 595) |
| 0,0023 g | Basic Violett 14 (C. I. 42 510) |
| 46,94 g | Wasser |
| 50,00 g | Ethanol |
| 100,00 g | |
| 8,1 | pH-Wert |

**Beispiel 28: Haarspray mit starker Festigung**

| 0,30 g | 4-Cascade: 1,4-Diaminobutan [4]: propylamin |
| --- | --- |
| 5,00 g | t-Octylacrylamid/Acrylsäure/t-Butylaminoethylmethacrylat Terpolymer |
| 0,58 g | 2-Amino-2-methyl-1-propanol |
| 0,15 g | Parfüm |
| 40,00 g | 1,1-Difluorethan |
| 53,97 g | Ethanol |
| 100,00 g | |
| 9,0 | pH-Wert (1:1 mit Wasser verdünnt) |

**Beispiel 29: Haarspray**

| 0,55 g | 4-Cascade: 1,4-Diaminobutan [4]: propylamin |
| --- | --- |
| 3,50 g | Vinylacetat/Crotonsäure/Vinylneodecanoat Terpolymer |
| 0,15 g | Parfüm |
| 0,14 g | Ameisensäure |
| 45,00 g | 1,1-Difluorethan |
| 50,66 g | Ethanol |
| 100,00 g | |
| 8,1 | pH-Wert (1:1 mit Wasser verdünnt) |

**Beispiel 30: Styling-Haarspray**

| 0,40 g | 32-Cascade:1,4-Diaminobutan[4]:(1-azabutyliden)[28]: propylamin |
| --- | --- |

(fortgesetzt)

| | |
|---|---|
| 6,50 g | Vinylpyrrolidon/Vinylacetat Copolymer |
| 0,17 g | Ameisensäure |
| 0,10 g | Parfüm |
| 10,67 g | Butan (1,5 bar) |
| 33,33 g | 1,1-Difluorethan |
| 42,83 g | Ethanol |
| 100,00 g | |
| 7,1 | pH-Wert (1:1 mit Wasser verdünnt) |

**Beispiel 31: 80 % VOC-Haarspray**

| | |
|---|---|
| 0,20 g | 4-Cascade: 1,4-Diaminobutan: propylamin |
| 4,00 g | t-Butylacrylat/Ethylacrylat/Methacrylsäure Terpolymer |
| 0,72 g | 2-Amino-2-methyl-1-propanol |
| 0,20 g | Cyclo-Tetra(dimethylsiloxan) |
| 0,05 g | Parfüm |
| 15,00 g | Wasser |
| 9,83 g | Ethanol |
| 40,00 g | Dimethylether |
| 100,00 g | |
| 9,1 | pH-Wert (1:1 mit Wasser verdünnt) |

**Beispiel 32: Pumpspray**

| | |
|---|---|
| 0,27 g | 4-Cascade: 1,4-Diaminobutan: propylamin |
| 4,50 g | t-Octylacrylamid/Acrylsäure/t-Butylamino-ethylmethacrylat Terpolymer |
| 0,52 g | 2-Amino-2-methyl-1-propanol |
| 0,30 g | Parfüm |
| 0,10 g | Dimethylsiloxan/Ethylenglykol Copolymer |
| 6,31 g | Wasser |
| 88,00 g | Ethanol |
| 100,00 g | |
| 8,9 | pH-Wert (1:1 mit Wasser verdünnt) |

**Beispiel 33: 80 % VOC-Pumpspray**

| | |
|---|---|
| 0,60 g | 64-Cascade: 1,4-Diaminobutan:(1-azabutyliden)[60]: propylamin |
| 5,00 g | Vinylpyrrolidon/Vinylacetat Copolymer |
| 0,30 g | Parfüm |
| 0,26 g | Ameisensäure |
| 13,84 g | Wasser |
| 80,00 g | Ethanol |
| 100,00 g | |
| 5,7 | pH-Wert (1:1 mit Wasser verdünnt) |

**Beispiel 34: 55 % VOC-Pumpspray**

| | |
|---|---|
| 0,79 g | 8-Cascade: 1,4-Diaminobutan:(1-aza-butyliden)$_4$:propylamin |
| 3,50 g | Vinylacetat/Crotonsäure Copolymer |
| 0,28 g | Ameisensäure |
| 0,20 g | Parfüm |
| 40,23 g | Wasser |
| 55,00 g | Ethanol |
| 100,00 g | |
| 7,7 | pH-Wert |

**Beispiel 35: Haargel**

| | |
|---|---|
| 0,60 g | 16-Cascade: 1,4-Diaminobutan:(1-aza-butyliden)$_{12}$: propylamin |
| 2,50 g | Hydroxypropylmethylcellulose |
| 0,80 g | Polyoxyethylen-(20)-sorbitanmonopalmitat |
| 0,50 g | Polyoxyethylen-(25)-p-Aminobenzoesäure |
| 0,40 g | Ameisensäure |
| 0,12 g | Cis-1-(3-Chlorallyl)-3,5,7-triaza-1-azonia-adamantanchlorid |
| 0,10 g | Parfüm |
| 23,00 g | Glycerin (86prozentig) |
| 71,98 g | Wasser |
| 100,00 g | |
| 5,4 | pH-Wert |

**Beispiel 36: Festigendes Haarstylinggel**

| | |
|---|---|
| 0,40 g | 32-Cascade: 1,4-Diaminobutan:(1-azabutyliden)$_{28}$: propylamin |
| 2,50 g | Polyvinylpyrrolidon |
| 2,00 g | Hydroxypropyl-Guar |
| 0,80 g | Hydriertes Rhizinusöl, oxethyliert mit 45 Mol Ethylenoxid |
| 0,60 g | DL-2-Pyrrolidon-S-carbonsäure |
| 0,45 g | Natriumbenzoat |
| 0,30 g | Hydroxyethylcellulose |
| 0,20 g | Parfüm |
| 0,09 g | Natriumformiat |
| 0,05 g | Mica/Titanoxid/Zinnoxid-Pulver (Soloron Silver Sparkle der Firma Merck/Deutschland) |
| 92,61 g | Wasser |
| 100,00 g | |
| 6,6 | pH-Wert |

**Beispiel 37: Haarfestigendes Liquid-Gel**

| | |
|---|---|
| 1,00 g | 4-Cascade: 1,4-Diaminobutan [4]: propylamin |
| 3,00 g | Vinylpyrrolidon/Vinylacetat Copolymer |

(fortgesetzt)

| | |
|---|---|
| 1,80 g | Polyoxyethylen-(20)-sorbitanmonopalmitat |
| 1,35 g | Polyethylenglykol-(45) |
| 1,05 g | Hydroxyethylcellulose |
| 1,00 g | Zitronensäure |
| 0,20 g | 1,2-Dibrom-2,4-dicyanobutan |
| 0,20 g | Parfüm |
| 90,40 g | Wasser |
| 100,00 g | |
| 7,5 | pH-Wert |

Sämtliche angegebenen Prozentzahlen stellen Gewichtsprozente dar, sofern nichts anderes vermerkt ist.

**Beispiel 38: Vergleichsversuche zur Naß- und Trockenkämmbarkeit**

Es wurden vier erfindungsgemäße, wäßrige Lösungen A bis D mit fünf wäßrigen Lösungen E bis I von haarpflegenden Standardpolymeren hinsichtlich ihrer Wirkung auf Naß- und Trockenkämmbarkeit von mit diesen Lösungen behandelten Haaren untersucht:

| Muster | Polymer | Konzentration | pH-Wert |
|---|---|---|---|
| A | 4-Cascade: 1,4-Diaminobutan: propylamin | 1,50 % | 6,0 |
| B | 4-Cascade: 1,4-Diaminobutan: propylamin | 0,25 % | 3,5 |
| C | 64-Cascade: 1,4-Diaminobutan: (1-aza-butyliden)$^{60}$: propylamin | 0,25 % | 6,0 |
| D | 64-Cascade: 1,4-Diaminobutan: (1-aza-butyliden)$^{60}$: propylamin | 1,50 % | 3,5 |
| E | Vinylpyrrolidon/Dimethylaminoethylmethacrylat Copolymer | 0,25 % | 6,0 |
| F | Vinylpyrrolidon/Dimethylaminoethylmethacrylat Copolymer | 1,50 % | 3,5 |
| G | Vinylpyrrolidon/Dimethylaminoethylmethacrylat Copolymer | 0,25 % | 3,5 |
| H | Kationische Hydroxy-propylcellulose | 1,50 % | 6,0 |
| I | Kationisches Hydroxy-propylcellulose | 0,25 % | 3,5 |

Standardisierte Haarsträhnen einer einheitlichen Länge von 17 cm und einer Breite von 2,5 cm wurden zuerst mit Bleichpulver und 9prozentiger Wasserstoffperoxidlösung 30 Minuten lang gebleicht, dann 2 Minuten lang mit Wasser ausgespült, zweimal mit einem üblichen Reinigungsmittel gewaschen, erneut gespült und nach dem Trocknen auf ein einheitliches Gewicht gebracht.

Nach der Vorkämmung und dem Einstellen auf eine Restfeuchte von 50 % wurden je 0,5 ml der Polymerlösungen auf die einzelnen Strähnen gleichmäßig aufgetragen und in einer Folientasche einmassiert. Nach einer Einwirkzeit von 5 Minuten erfolgte die Messung der Kämmkraft für die Naßkämmung sowie nach dem Trocknen der Kämmsträhne die Messung der Kämmkraft für die Trockenkämmung.

Es wurden folgende Kämmkräfte gemessen:

| Kämmkraft [N] | | |
|---|---|---|
| Muster | Naßkänunung | Trockenkämmung |
| A | 0,279 | 0,141 |
| B | 0,261 | 0,122 |
| C | 0,284 | 8,251 |
| D | 0,257 | 0,217 |
| E | 0,371 | 0,261 |
| F | 0,313 | 0,399 |

(fortgesetzt)

| Kämmkraft [N] | | |
|---|---|---|
| Muster | Naßkänunung | Trockenkämmung |
| G | 0,387 | 0,235 |
| H | 0,390 | 0,252 |
| I | 0,407 | 0,254 |

Die mittlere Kämmkraft der unbehandelten Strähnen betrug 0,47 N. Die mit den erfindungsgemäßen Zusammensetzungen A bis D behandelten Strähnen wiesen eine deutliche Reduzierung der Kämmkraft auf.

**Patentansprüche**

1. Kosmetisches Mittel zur Haarbehandlung mit einem Gehalt an mindestens einem Dendrimer oder Dendrimerkonjugat in einer geeigneten kosmetischen Grundlage.

2. Mittel nach Anspruch 1, **dadurch gekennzeichnet, daß** es 0,01 bis 75 Gewichtsprozent mindestens eines Dendrimers oder Dendrimerkonjugats enthält.

3. Mittel nach Anspruch 1 oder 2, **dadurch gekennzeichnet, daß** es Dendrimere oder Dendrimerkonjugate auf der Basis von 1,4-Diaminobutan und Acrylnitril enthält.

4. Mittel nach einem der Ansprüche 1 bis 3, **dadurch gekennzeichnet, daß** es zusätzlich 0,01 bis 40 Gewichtsprozent mindestens eines anionischen, kationischen, amphoteren oder nicht-ionischen Tensids und 50 bis 90 Gewichtsprozent Wasser enthält.

5. Mittel nach einem der Ansprüche 1 bis 3, **dadurch gekennzeichnet, daß** es zusätzlich 0,05 bis 15 Gewichtsprozent mindestens einer keratinreduzierenden Mercaptoverbindung enthält.

6. Mittel nach einem der Ansprüche 1 bis 3, **dadurch gekennzeichnet, daß** es zusätzlich 0,05 bis 2 Gewichtsprozent mindestens eines direkt auf das Haar aufziehenden synthetischen oder natürlichen Haarfarbstoffs enthält.

7. Mittel nach einem der Ansprüche 1 bis 3, **dadurch gekennzeichnet, daß** es zusätzlich 0,01 bis 25 Gewichtsprozent mindestens eines synthetischen oder natürlichen Polymers enthält.

8. Verwendung von mindestens einem Dendrimer oder Dendrimerkonjugat in einem Blondiermittel oder in einem Oxidationshaarfärbemittel.

9. Verwendung von mindestens einem Dendrimer oder Dendrimerkonjugat in einem Haarpflegemittel.

**Claims**

1. Cosmetic agent for hair treatment, containing at least one dendrimer or dendrimer conjugate in a suitable cosmetic base.

2. Agent according to claim 1, **characterised in that** it contains from 0.01 to 75% by weight of at least one dendrimer or dendrimer conjugate.

3. Agent according to claim 1 or 2, **characterised in that** it contains dendrimers or dendrimer conjugates based on 1,4-diaminobutane and acrylonitrile.

4. Agent according to any one of claims 1 to 3,
**characterised in that** it additionally contains from 0.01 to 40% by weight of at least one anionic, cationic, amphoteric or nonionic surfactant and from 50 to 90% by weight of water.

5. Agent according to any one of claims 1 to 3,
   **characterised in that** it additionally contains from 0.05 to 15% by weight of at least one keratin-reducing mercapto compound.

6. Agent according to any one of claims 1 to 3,
   **characterised in that** it additionally contains from 0.05 to 2% by weight of at least one synthetic or natural direct hair dye.

7. Agent according to any one of claims 1 to 3,
   **characterised in that** it additionally contains from 0.01 to 25% by weight of at least one synthetic or natural polymer.

8. Use of at least one dendrimer or dendrimer conjugate in a blonding agent or in an oxidative hair colouring agent.

9. Use of at least one dendrimer or dendrimer conjugate in a hair care agent.


**Revendications**

1. Agent cosmétique pour le traitement des cheveux, présentant une teneur en au moins un dendrimère ou un conjugat de dendrimère, dans une base cosmétique appropriée.

2. Agent selon la revendication 1, **caractérisé en ce qu'**il contient de 0,01 à 75 pour cent en poids d'au moins un dendrimère ou un conjugat de dendrimère.

3. Agent selon la revendication 1 ou 2, **caractérisé en ce qu'**il contient un dendrimère ou un conjugat de dendrimère à base de 1,4-diaminobutane et d'acrylonitrile.

4. Agent selon l'une des revendications 1 à 3, **caractérisé en ce que**, en plus, il contient de 0,01 à 40 pour cent en poids d'au moins un tensio-actif anionique, cationique, amphotère ou nonionique et 50 à 90 pour cent en poids d'eau.

5. Agent selon l'une des revendications 1 à 3, **caractérisé en ce qu'**il contient en plus de 0,05 à 15 pour cent en poids d'au moins un composé mercapto réducteur de la kératine.

6. Agent selon l'une des revendications 1 à 3, **caractérisé en ce qu'**il contient en plus de 0,05 à 2 pour cent en poids d'au moins un colorant capillaire synthétique ou naturel à appliquer directement sur les cheveux.

7. Agent selon l'une des revendications 1 à 3, **caractérisé en ce qu'**il contient en plus de 0,01 à 25 pour cent en poids d'au moins un polymère synthétique ou naturel.

8. Utilisation d'au moins un dendrimère ou d'un conjugat de dendrimère dans un agent de décoloration, ou bien dans un agent de teinture capillaire travaillant par oxydation.

9. Utilisation d'au moins un dendrimère ou d'un conjugat de dendrimère dans un agent de soin capillaire.